# EUROPÄISCHE PATENTSCHRIFT

(11) **EP 1 010 002 B1**
(45) Veröffentlichungstag und Bekanntmachung des Hinweises auf die Patenterteilung: **12.12.2001**
(21) Anmeldenummer: 99924753.9
(22) Anmeldetag: 01.04.1999
(51) Int. Cl.: G01N 27/12, G01N 33/00

(54) **WASSERSTOFFSENSOR**
HYDROGEN SENSOR
DETECTEUR POUR HYDROGENE

(30) Priorität: 30.04.1998 DE 19819575
(43) Veröffentlichungstag der Anmeldung: 21.06.2000
(73) Patentinhaber: SIEMENS AKTIENGESELLSCHAFT, 80333 München (DE)
(72) Erfinder: JONDA, Sven, D-85567 Alxing (DE); MEIXNER, Hans, D-85540 Haar (DE)
(86) Internationale Anmeldenummer: DE9901003
(87) Internationale Veröffentlichungsnummer: WO9957548

(56) Entgegenhaltungen:
- EP-A- 0 603 945
- EP-A- 0 798 554
- DE-C- 4 203 522

## Beschreibung

Die vorliegende Erfindung betrifft einen Wasserstoffsensor mit einer temperaturabhängig wasserstoffempfindlichen Halbleiterschicht und einer selektiv wasserstoffdurchlässigen Deckschicht.

Bei Gassensoren besteht ein allgemeines Problem darin, daß Veränderungen des Meßwertes auch dann beobachtet werden, wenn sich die zu messende Gaskomponente selbst nicht ändert. Dies ist besonders dann störend, wenn eine Komponente mit niedrigem Mischungsverhältnis bzw. niedriger Konzentration in Anwesenheit großer, aber variabler Konzentrationen von Querempfindlichkeiten verursachenden Gasen gemessen werden soll.

Es ist versucht worden, einen Wasserstoffsensor zu entwikkeln, der mit Galliumoxid, Ga₂O₃, als halbleitendem Metalloxid arbeitet. Diese Substanz spricht auch auf andere Gase, insbesondere Sauerstoff und Kohlenwasserstoffe an. Der Sensor ist damit für Messungen in Umgebungen, in welchen sich die genannten Gaskomponenten stark ändern, allenfalls bedingt geeignet. Es ist daher versucht worden, eine Deckschicht über dem Galliumoxid anzuordnen, welche selektiv Wasserstoff durchläßt, während andere Komponenten am Zutritt zu der gasempfindlichen Halbleiterschicht gehindert werden.

Auch mit einer solchen Deckschicht liefert ein Galliumoxidsensor aber oftmals nur unbefriedigende Ergebnisse bei der Abgasmessung. Neben der Anwesenheit von störenden Gaskomponenten in schwankender Zusammensetzung wirken sich nämlich auch andere Faktoren bei der Gasmessung, insbesondere der Abgasmessung in Automobilen negativ aus. Hierzu zählt, daß durch elektromagnetische Einstreuungen usw., wie sie im Automobilbereich besonders stark auftreten können, die Messung der elektrischen Parameter wie der elektrischen Leitfähigkeit der eingesetzten Halbleiterschichten oft sehr schlecht sind. Zudem kann sich auch die Variation anderer Größen negativ auswirken, etwa der Temperatur des eingesetzten Sensors durch Umgebungstemperaturschwankungen oder durch Driften der Heizungsenergiezufuhr.

In der DE 42 03 522 C1 wird eine Sauerstoffsensoranordnung auf der Basis halbleitender Metalloxide beschrieben, in welcher die Temperaturempfindlichkeit reduziert ist. Die Leitfähigkeit bei erhöhter Temperatur der Metalloxide hängt vom Sauerstoffpartialdruck ab, wobei die Sensoranordnung zwei Metalloxid-Einzelsensoren aufweist, die im beabsichtigten Meßbereich eine unterschiedliche Abhängigkeit der Leitfähigkeit vom Sauerstoffpartialdruck, hingegen eine weitgehend gleiche Temperaturabhängigkeit der Leitfähigkeit zeigen. Die Temperaturabhängigkeit soll sich im gebildeten Quotienten der Leitfähigkeitsmeßsignale beider Sensoren entsprechend weitgehend herausheben.

Die vorliegende Erfindung zielt darauf, Neues für die gewerbliche Anwendung bereitzustellen, insbesondere, jedoch nicht ausschließlich, einen Gassensor zu schaffen, der zur Wasserstoffmessung besonders geeignet ist und der insbesondere auch zu Abgasmessungen eingesetzt werden kann.

Die Lösung der Aufgabe wird unabhängig beansprucht, bevorzugte Ausführungsformen finden sich in den abhängigen Ansprüchen.

Die Erfindung beruht auf der Erkenntnis, daß Strontiumtitanat nach Abdeckung mit einer selektiv Wasserstoff filternden Deckschicht hervorragend zum Einsatz als Wasserstoffsensor geeignet ist und daß durch eine geeignete unterschiedliche Dotierung zweier Schichten auch bei der Wasserstoffmessung eine Kompensation der Temperaturempfindlichkeit erreicht werden kann. Es wird davon ausgegangen, daß die Filterwirkung für Wasserstoff hervorgerufen wird durch eine selektive Permeabilität für diese Substanz, aber auch eine insbesondere quasiselektive Umsetzung von Wasserstoff mit oxidierenden Substanzen in der Deckschicht kann nicht vollständig ausgeschlossen werden. Auch der genaue Wirkungsmechanismus der Leitfähigkeitsänderung ist unklar. Es wird einerseits vermutet, daß, anders als im Fall des Sauerstoffnachweises mit Strontiumtitanat-Halbleitern, wo Sauerstoff in atomarer Form in das Kristallgitter eingebracht wird und dort die elektronischen Eigenschaften desselben verändert, vorliegend eine chemische reversible Reaktion zwischen dem Wasserstoff und den Materialien im Halbleiter erfolgt, welche nur indirekt als Änderung einer elektrischen Größe erfassbar ist. Eine weitere Vermutung ist, daß sich Wasserstoff an der Oberfläche des Strontiumtitanats anlagert und dadurch die elektronische Bandstruktur des Halbleiters nahe der Oberfläche ändert.

Eine bevorzugte Deckschicht wird Silizium als Bestandteil enthalten, was darauf zurückgeführt wird, daß viele Siliziumverbindungen ein so enges Kristallgitter besitzen, daß außer Wasserstoff keine oder keine relevanten Gase passieren können, und weiter vorteilhaft ist, viele Siliziumverbindungen im vorgesehenen Temperaturbereich der Sensoren allenfalls schlecht leiten, so daß die Messungen der elektrischen Parameter der Strontiumtitanat-Halbleiterschichten allenfalls wenig und praktisch nicht signifikant beeinflußt werden. Bevorzugt bestehen die Deckschichten aus Siliziumdioxid und/oder Siliziumnitrid. Die Auftragung der Deckschicht als Dünnschicht durch Sputtern bzw. CVD stellt eine Deckschicht-Struktur sicher, welche für störende Gase hinreichend unpassierbar ist.

Bevorzugt wird eine der Strontiumtitanatschichten n- und die zweite p-dotiert sein. Die durch Wasserstoff hervorgerufenen Eigenschaftsänderungen im Strontiumtitanat sind reversibel und können sich durch unterschiedliche dominierende Mechanismen der elektrischen Leitung in den n- und p-dotierten Schichten typisch als entgegengesetzte Leitfähigkeitsänderungen der Einzelschichten auswirken. So wird in einer Schicht eine Leitfähigkeitsabnahme und in der anderen Schicht eine Leitfähigkeitszunahme beobachtet.

Es ist bevorzugt, die elektrische Leitfähigkeit beider Halbleiterschichten in Abhängigkeit von der Wasserstoffkonzentration zu messen und dann die Differenz bzw. den Quotient der jeweiligen Meßwerte als Wasserstoff-Meßsignal zu bestimmen.

Die Erfindung wird im folgenden nur beispielsweise anhand der Zeichnung beschrieben. In dieser zeigt:

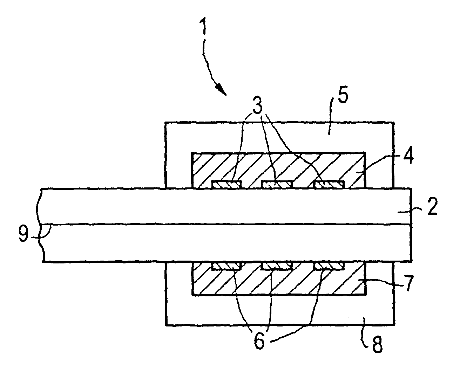

- Figur 1: einen Wasserstoffsensor nach der vorliegenden Erfindung.

Nach Figur umfaßt ein allgemein mit 1 bezeichneter Wasserstoffsensor ein Trägersubstrat 2 aus elektrisch isolierendem, hochtemperaturstabilem Material wie Al₂O₃. Auf der einen Seite des Trägersubstrates 2 ist über einer interdigitalen Elektrodenstruktur 3 eine Strontiumtitanatschicht 4 aufgebracht, die vollständig von einer Deckschicht 5 überdeckt ist. Auf der gegenüberliegenden Seite des Trägersubstrates 2 ist in spiegelbildlicher Weise eine zweite interdigitale Elektrodenstruktur 6 vorgesehen, über welcher eine zweite Strontiumtitanatschicht 7 angeordnet ist, die allseitig von einer Deckschicht 8 umgeben ist.

Das Trägersubstrat 2 kann als bei hoher Temperatur gemeinsam gebrannte Keramikstruktur (HTTC, High temperature cofired ceramic) gebildet sein und im Inneren eine Temperatursensoranordnung und/oder eine Heizungsmäanderanordnung umfassen, wie in der Zeichnung gemeinsam durch die Bezugszahl 9 veranschaulicht. Bevorzugt ist die Heizungsmäanderanordnung zentral vorgesehen, um beide Strontiumtitanatschichten 4 und 7 bzw. beide Deckschichten 5 und 8 auf dieselbe Temperatur zu erwärmen und dafür ausgelegt, dort Temperaturen zwischen 800° und 1000° C zu erzeugen.

Die Strontiumtitanatschichten 4 und 7 unterscheiden sich in ihren Halbleitereigenschaften. In der einen Strontiumtitanatschicht dominiert die p-Leitung, in der anderen die n-Leitung. Dies wird durch eine geeignete Dotierung und eine hinreichend hohe Konzentration an Sauerstoff-Fehlstellen im Strontiumtitanat erreicht. Die Strontiumtitanatschicht 4 ist daher n-dotiert, während die Strontiumtitanatschicht 7 p-dotiert ist. Als geeigneten Materialien zur Dotierung sind Cr³⁺-Ionen zur Akzeptordotierung geeignet, die Ti⁴⁺ durch die identischen Ionenradien von 61 pm besonders gut substituieren, während als Donatoren Ta⁵⁺-Ionen verwendbar sind. Die erforderliche Sauerstoff-Fehlstellenkonzentration wird erzielt, indem die gebildeten Schichten in einer Atmosphäre mit Sauerstoffkonzentrationen von wenigstens 1 % getempert bzw. gesintert werden. Danach ist die Sauerstoff-Fehlstellenkonzentration des Strontiumtitanats so höch, daß der dominierende Leitungsmechanismus der einen Schicht die n-Leitung und der anderen Schicht die p-Leitung ist.

Die interdigitalen Elektrodenstrukturen 3 und 6 aus geeignetem Metall wie Platin oder einer Platin-Legierung befinden sich in elektrisch leitendem Kontakt mit jeweiligen Strontiumtitanatschichten und weisen zur Bestimmung der Strontiumtitanat-Leitfähigkeiten Anschlußfelder auf, die mit einer Auswerteschaltung verbunden werden können.

Die Deckschichten 5 und 8 werden bevorzugt identisch aufgebaut und bestehen aus einem Material, welches selektiv Wasserstoff durchläßt. Geeignet sind insbesondere Siliziumverbindungen wie Siliziumdioxid oder Siliziumnitrid.

Der Gassensor der vorliegenden Erfindung wird wie folgt betrieben:
Der Gassensor wird an dem Verwendungsort, wie einem Abgaskanal einer Feuerungsanlage, eines Verbrennungsmotors usw. eingebaut und die Heizungsstruktur und/oder der Temperatursensor 9 sowie die interdigitalen Elektrodenstrukturen 3 bzw. 6 werden an eine entsprechende Beschaltung angeschlossen. Die Beschaltung ist dazu ausgelegt, durch die Heizungsstruktur einen Strom vorzusehen, mit welchem der relevante Bereich des Wasserstoffsensors 1 auf eine Temperatur von etwa 800° bis 1000° C erwärmt wird.

Die interdigitalen Elektrodenstrukturen 3 bzw. 6 werden angeschlossen, um die elektrische Leitfähigkeit der Strontiumtitanatschichten 4 bzw. 7 zu messen.

Wenn sich in der Umgebung des Wasserstoffsensors 1 die Konzentration an Wasserstoffgas ändert, ändert sich die elektrische Leitfähigkeit des Sensors.

Die genaue Ursache hierfür ist nicht vollständig verstanden. Es wird aber vermutet, daß sich über die Deckschicht ein Wasserstoff-Gradient ausbildet und eine Wanderung von Wasserstoff in die eine oder andere Richtung durch die Deckschicht 5 bzw. 8 erfolgen. Dies kann durch selektive Permeabilität der Deckschicht für Wasserstoff oder durch Abreaktion des Wasserstoffes mit anderen Substanzen in der Deckschicht bewirkt sein.

In beiden Strontiumtitanatschichten 4 bzw. 7 ändert sich im Ansprechen darauf die elektrische Leitfähigkeit, und zwar auf jeweils unterschiedliche Weise. Auch hierfür ist der Mechanismus nicht vollständig klar. Eine mögliche Ursache stellen chemische Reaktionen zwischen dem Strontiumtitanat und dem in dieses eindringenden Wasserstoff dar; die chemischen Reaktionen führen wiederum zu einer Änderung der Sauerstoffleerstellenkonzentration im Strontiumtitanat. In beiden Schichten tritt dann je nach Änderung der Wasserstoffkonzentration entweder eine Sauerstoffzunahme oder eine Sauerstoffabnahme auf. Da durch den Herstellungsprozeß die intrinsische Sauerstoffleerstellenkonzentration beider Strontiumtitanatschichten aber derart unterschiedlich ist, daß in der einen Strontiumtitanatschicht bei der gegebenen Dotierung der dominierende Leitungsmechanismus die p-Leitung ist und bei der anderen Strontiumtitanatschicht mit der anderen Dotierung der dominierende Leitungsmechanismus die n-Leitung ist, ändern sich hierdurch die elektrischen Leitfähigkeiten beider Strontiumtitanatschichten auf entgegengesetzte Weise. Während sich bei der einen Strontiumtitanatschicht mit steigenden Wasserstoffkonzentrationen eine abnehmende Leitfähigkeit ergibt, steigt gleichzeitig die elektrische Leitfähigkeit der anderen Strontiumtitanatschicht. Dennoch ändert sich aufgrund der von der Dotierung allenfalls wenig beeinflußten thermischen Aktivierungsenergie der Strontiumtitanatschichten die elektrische Leitfähigkeit beider Schichten mit der Temperatur auf gleiche Weise.

Eine weitere Erklärung ist die Anlagerung von Wasserstoff an die Oberfläche, was Änderungen der Bandstruktur hervorrufen könnte und ebenfalls ein geeignetes Modell zur Erklärung der beobachteten Effekte darstellt.

Die Leitfähigkeit wird mit den interdigitalen Elektrodenstrukturen 3 und 6 abgetastet und in einer Auswerteschaltung ausgewertet. Dort wird der Quotient der beiden Leitfähigkeiten bestimmt. Da sich beide Leitfähigkeiten zwar auf unterschiedliche Weise mit der Wasserstoffkonzentration ändern, aber die wenigstens näherungsweise exponentielle Temperaturabhängigkeit bei beiden Schichten insbesondere unterhalb von 800°C praktisch gleich ist und auch darüber kaum abweicht, wird ein weitgehend temperaturunabhängiges Maß für die Wasserstoffkonzentration erhalten.

So ist es möglich, mit der Auswerteschaltung durch Quotientenbildung der an beiden Schichten gewonnenen jeweiligen elektrischen Leitfähigkeiten ein temperaturunabhängiges Signal mit großer Amplitude zu gewinnen.

Anstelle einer gegenüberliegenden Anordnung beider Sensorbereiche können diese auch nebeneinander angeordnet werden.

## Patentansprüche

1. Wasserstoffsensor (1) mit einer temperaturabhängig wasserstoffempfindlichen Halbleiterschicht (4) und einer selektiv wasserstoffdurchlässigen Deckschicht (5), **dadurch gekennzeichnet, daß** die Halbleiterschicht (4) aus Strontiumtitanat, SrTiO₃ besteht, der zur Kompensation ihrer Temperaturempfindlichkeit eine weitere Strontiumtitanatschicht (7) zugeordnet ist, welche von einer selektiv Wasserstoff filternden Deckschicht (8) abgedeckt ist und welche einen anderen Verlauf der Leitfähigkeitskennlinie als die erste Halbleiterschicht aufweist.

2. Wasserstoffsensor nach dem vorhergehenden Anspruch, worin die Deckschicht mit Silizium oder mit einer Siliziumverbindung aufgebaut ist.

3. Wasserstoffsensor nach dem vorhergehenden Anspruch, worin die Deckschicht aus Siliziumdioxid, SiO₂, oder Siliziumnitrid, Si₃N₄, besteht.

4. Wasserstoffsensor nach einem der vorhergehenden Ansprüche, worin die Deckschicht als Dünnschicht aufgetragen ist.

5. Wasserstoffsensor nach dem vorhergehenden Anspruch, worin die Deckschicht durch Sputtern oder in CVD (chemical vapor deposition) hergestellt ist.

6. Wasserstoffsensor nach einem der vorhergehenden Ansprüche, worin die erste Strontiumtitanatschicht n- und die zweite p-dotiert ist.

7. Wasserstoffsensor nach einem der vorhergehenden Ansprüche, worin die Strontiumtitanatschichten in Sauerstoffatmosphäre gesintert und/oder getempert sind.

8. Wasserstoffsensoranordnung mit einem Wasserstoffsensor nach einem der vorhergehenden Ansprüche und einer Auswerteschaltung zur Messung wenigstens eines elektrischen Parameters der beiden Strontiumtitanatschichten, insbesondere zur Messung der jeweiligen elektrischen Leitfähigkeit, sowie zur Berechnung der Differenz und/oder des Quotienten der jeweiligen Messwerte.

## Claims

1. Hydrogen sensor (1) having a semiconductor layer (4) which is sensitive to hydrogen as a function of temperature and a covering layer (5) which is selectively permeable to hydrogen, **characterized in that** the semiconductor layer (4) consists of strontium titanate, SrTiO₃, which, in order to compensate for its temperature sensitivity, is assigned a further strontium titanate layer (7) which is covered by a covering layer (8) which selectively filters hydrogen and which has a different conductivity characteristic profile from the first semiconductor layer.

2. Hydrogen sensor according to the preceding claim, in which the covering layer is built up of silicon or a silicon compound.

3. Hydrogen sensor according to the preceding claim, in which the covering layer consists of silicon dioxide, SiO₂, or silicon nitride, Si₃N₄.

4. Hydrogen sensor according to one of the preceding claims, in which the covering layer is applied as a thin film.

5. Hydrogen sensor according to the preceding claim, in which the covering layer is produced by sputtering or CVD (chemical vapour deposition).

6. Hydrogen sensor according to one of the preceding claims, in which the first strontium titanate layer is n-doped and the second is p-doped.

7. Hydrogen sensor according to one of the preceding claims, in which the strontium titanate layers are sintered and/or annealed in an oxygen atmosphere.

8. Hydrogen sensor arrangement having a hydrogen sensor according to one of the preceding claims and an evaluation circuit for measuring at least one electrical parameter of the two strontium titanate layers, in particular for measuring the corresponding electrical conductivity, and for calculating the difference and/or the quotient from the corresponding measured values.

## Revendications

1. Détecteur (1) d'hydrogène comprenant une couche (4) semi-conductrice sensible à l'hydrogène en fonction de la température et une couche (5) de finition perméable sélectivement à l'hydrogène, **caractérisé en ce que** la couche (4) semi-conductrice est en titanate de strontium, SrTiO₃, et il lui est associé, pour compenser sa sensibilité à la température, une autre couche (7) en titanate de strontium qui est recouverte d'une couche (8) de finition filtrant sélectivement l'hydrogène et qui a une autre courbe caractéristique de la conductivité que la première couche semi-conductrice.

2. Détecteur d'hydrogène suivant la revendication précédente, dans lequel la couche de finition est constituée de silicium ou d'un composé du silicium.

3. Détecteur d'hydrogène suivant la revendication précédente, dans lequel la couche de finition est en dioxyde de silicium, SiO₂, ou en nitrure de silicium, Si₃N₄.

4. Détecteur d'hydrogène suivant l'une des revendications précédentes, dans lequel la couche de finition est déposée sous la forme d'une couche mince.

5. Détecteur d'hydrogène suivant la revendication précédente, dans lequel la couche de finition est fabriquée par pulvérisation cathodique ou en CVD (chemical vapor deposition).

6. Détecteur d'hydrogène suivant l'une des revendications précédentes, dans lequel la première couche de titanate de strontium est à dopage n et la seconde à dopage p.

7. Détecteur d'hydrogène suivant l'une des revendications précédentes, dans lequel les couches de titanate de strontium sont frittées et/ou recuites en atmosphère d'oxygène.

8. Dispositif à détecteur d'hydrogène comprenant un détecteur d'hydrogène suivant l'une des revendications précédentes et un circuit d'exploitation destiné à mesurer au moins un paramètre électrique des deux couches de titanate de strontium, notamment à mesurer leur conductivité électrique, ainsi qu'à calculer la différence et/ou le quotient des valeurs mesurées respectives.
